# EUROPÄISCHE PATENTANMELDUNG

(11) **EP 4 838 721 A1**
(43) Veröffentlichungstag der Anmeldung: **11.11.2026**
(21) Anmeldenummer: 26175210.9
(22) Anmeldetag: 28.04.2026
(51) Int. Cl.: H01T 23/00, B05B 1/00, H01T 19/04, H05F 3/04

(54) **VORRICHTUNG ZUM IONISIEREN VON LUFT**

(30) Priorität: 05.05.2025 DE 202025102452 U
(71) Anmelder: Herrmann AG, 93483 Pösing (DE)
(72) Erfinder: Herrmann, Johannes, 93483 Pösing (DE)
(74) Vertreter: Hannke Bittner & Partner mbB Regensburg

(57) **Zusammenfassung**

Vorrichtung (1) zum Beaufschlagen von Oberflächen und insbesondere von Kraftfahrzeugoberflächen mit ionisierter Luft mit einer Zuführanschluss, um der Vorrichtung (1) Druckluft zuzuführen, mit einer Ionisierungseinheit (2), welche dazu geeignet und bestimmt ist, wenigstens einen Anteil der über den Zuführanschluss (2) zugeführten Luft zu ionisieren und mit einer Ausgabeeinrichtung (6), welche die ionisierte Luft auf die Oberfläche ausgibt, wobei die Ionisierungseinheit (2) eine Energiewandlereinrichtung (22) aufweist, welche eine Bewegungsenergie des Luftstroms wenigstens teilweise in eine elektrische Energie umwandelt, wobei die Vorrichtung (1) einen Griffabschnitt (42) aufweist, welcher zum Halten der Vorrichtung dient sowie einen Ausgabeabschnitt (44), der zum Ausgeben der ionisierten Luft dient,
dadurch gekennzeichnet, dass
die Ionisiereinheit (2) dazu geeignet und bestimmt ist, die Luft mit einer zeitlich variierenden Spannung zu ionisieren.

## Beschreibung

Die vorliegende Erfindung bezieht sich auf eine Vorrichtung zum Ionisieren von Luft. In der Kraftfahrzeugtechnik ist es bekannt, dass etwa zu lackierende Oberflächen vor der Lackierung mit ionisierter Luft beaufschlagt werden. Auf diese Weise können insbesondere Staubreste auf den besagten zu lackierenden Oberflächen entfernt werden. Aus dem Stand der Technik sind zu diesem Zweck unterschiedliche Beaufschlagungseinrichtungen bekannt, mit denen ionisierte Luft auf die Fahrzeugoberflächen aufgebracht werden kann. Üblicherweise sind dabei etwa Luftdruckpistolen an Hochdruckleitungen angeschlossen und geben so Luft aus. Daneben sind diese Pistolen üblicherweise an ein Stromnetz angeschlossen, welches die Luftdruckpistole mit Spannung und insbesondere Hochspannung versorgt, um die ionisierte Luft zu erzeugen.

Weiterhin ist es aus dem internen Stand der Technik der Anmelderin bekannt, dass die zu beaufschlagenden Oberflächen ebenfalls elektrostatisch aufgeladen werden. Dies kann beispielsweise durch ein Reinigen bzw. Abwischen der Oberfläche auftrefen. Hierbei tritt das Problem auf, dass der Nutzer wissen muss, wie die Oberfläche aufgeladen ist, um entsprechend auch bei der Ionisierung der Luft die korrekte (nämlich die gleiche) Ladung zu verwenden.

Aus der DE 10 2021 109 607 A1 ist eine Vorrichtung zum Ionisieren von Druckluft bekannt.

Der vorliegenden Erfindung liegt daher die Aufgabe zugrunde, die hier beschriebenen Vorrichtungen bedienerfreundlicher zu gestalten.

Dies wird erfindungsgemäß durch die Gegenstände der unabhängigen Schutzansprüche erreicht. Vorteilhafte Ausführungsformen und Weiterbildungen sind Gegenstand der Unteransprüche

Bekannt sind metallische lonisatoren, welche mit Hochspannung versorgte leitfähige Spitzen aufweisen, die durch Koronaentladung und Feldemission in der unmittelbaren Umgebung Ionen erzeugen. Es gibt Ausführungen, die ungeregelt ständig Ionen erzeugen, und andere, bei denen das elektrische Feld durch Messung und gezielte Nachstellung der Hochspannung geregelt wird (geregelter Ionisator).

Geregelte und ungeregelte lonisatoren werden sowohl zum Aufbau definierter Ladungen als auch zur Ableitung unerwünschter Ladungen (Verhinderung von elektrostatischen Aufladungen und ESD) eingesetzt.

Um zu erreichen, dass die lonisatoren in größerem Abstand zum zu entladenden Material aufgestellt werden können (bis zu ca. 2 m), werden sie auch mit Blasluftdüsen ausgestattet oder an vorhandene Düsen angebaut. Dadurch werden die Ionen zusammen mit der Luft zur Wirkstelle hingeblasen (z. B. in Druckmaschinen).

Sägezahn-Ionisatoren sind oft als dünne kammartige Leisten ausgebildet. Der Begriff "Sägezahn" bezieht sich hier auf die geometrische Ausgestaltung der Leisten. Sie werden mit Gleich- oder Wechselspannung gespeist. Bei Wechselspannung werden alle Kammspitzen gemeinsam an die Spannungsquelle angeschlossen. Bei Gleichspannung sind die dicht nebeneinander angeordneten Metallspitzen abwechselnd positiv und negativ geladen.

Aus dem Stand der Technik sind daher lonisiereinrichtungen bekannt geworden, welche einen Batteriebetrieb aufweisen, wobei diese Batterie wiederum mit entsprechenden elektronischen Schaltungen eine Hochspannung erzeugt und diese Hochspannung zum Ionisieren der Luft verwendet wird. Diese Vorgehensweise hat einerseits den Nachteil, dass die Batterien in den Aggregaten relativ schwer sind. Andererseits müssen derartige Batterien auch regelmäßig aufgeladen werden, was wiederum zu Ausfall- und Standzeiten der Vorrichtung führt.

Aus der DE 20 2018 101 625 ist eine Ionisierungseinrichtung bekannt, welche zum Erzeugen der Spannung zum Ionisieren der Luft einen Generator verwendet, der von der zugeführten Druckluft angetrieben wird. Diese Ionisierungseinrichtung ist jedoch relativ umständlich in der Handhabung.

Es soll eine Vorrichtung geschaffen werden, die im Vergleich zu aus dem Stand der Technik bekannten Vorrichtungen bequemer in der Handhabung ist. Diese Aufgaben werden erfindungsgemäß durch den Gegenstand des unabhängigen Anspruchs erreicht. Vorteilhafte Ausführungsformen und Weiterbildungen sind Gegenstand der Unteransprüche.

Eine erfindungsgemäße Vorrichtung zum Beaufschlagen von Oberflächen und insbesondere von Kraftfahrzeugoberflächen mit ionisierter Luft weist einen Zuführanschluss auf, um der Vorrichtung Druckluft zuzuführen. Weiterhin weist die Vorrichtung eine Ionisierungseinheit auf, welche dazu geeignet und bestimmt ist, wenigstens einen Anteil der über den Zuführanschluss zugeführten Luft zu ionisieren. Daneben weist die Vorrichtung eine Ausgabeeinrichtung auf, welche ionisierte Luft ausgibt und insbesondere an eine (insbesondere elektrostatisch aufgeladene) Oberfläche ausgibt. Bei den Oberflächen kann es sich jedoch auch um Wände, Möbeloberflächen und generell um Oberflächen aller Art handeln. Besonders bevorzugt handelt es sich bei der Oberfläche um eine lackierte oder zu lackierende Oberfläche eines Kraftfahrzeugs.

Weiterhin weist die Ionisierungseinheit eine Energiewandlereinrichtung auf, welche eine Bewegungsenergie des Luftstroms wenigstens teilweise in eine elektrische Energie umwandelt.

Erfindungsgemäß die Ionisiereinheit dazu geeignet und bestimmt ist, die (durch diese strömende) Luft mit einem zeitlich variierenden Spannungspotential und/oder mit bzw. mittels einer zeitlich variierenden Spannung zu ionisieren.

Die zu beaufschlagenden Oberflächen werden teilweise mit einer vorgegebenen Ladung versehen. Dies kann beispielsweise beim Abwischen der Oberfläche mit einem Tuch auftreten. Die Anmelderin hat herausgefunden dass es bei Verwendung eines variierenden Spannungspotentials (und insbesondere eines zeitweise Positiven und zeitweise negativen Potenzials) der zu ionisierenden Luft bzw. der ionisierenden Spannung möglich ist, dass unabhängig von der Ladung der zu beaufschlagenden Oberfläche diese wirkungsvoll mit ionisierter Luft beaufschlagt werden kann (und auf diese insbesondere entladen werden kann).

Bevorzugt bewirkt daher die Vorrichtung, dass die Luft mit wechselnder Ladung ionisiert wird und beispielsweise zeitweise positiv ionisierte Luft und zweitweise negativ ionisierte Luft aus dieser austritt.

Bevorzugt wird die erzeugte Ionisierte Luft ebenfalls mit einem sich ändernden Potential und/oder einer sich ändernden Spannung ionisiert, also zeitweise positiv und zeitweise negativ. Die Anmelderin hat überraschenderweise festgestellt, dass auch der jeweilige Anteil mit dem richtigen Potential ausreichend zur Behandlung der jeweiligen Oberfläche ist.

Bei einer bevorzugten Ausführungsform ist die Ionisierungseinheit dazu geeignet und bestimmt ist die Luft wenigstens zeitweise mit eine positiven Spannung und/oder einem positiven Spannungpotential und wenigstens zeitweise mit einer negativen Spannung und/oder einem negativen Spannungspotential zu ionisieren. Bevorzugt wird die Luft mit einer insbesondere schwankenden Spannung ionisiert und/oder einem insbesondere periodisch schwankenden Spannungspotential beaufschlagt.

Bevorzugt weist die Vorrichtung und/oder die Ionisierungseinheit eine Wechselspannungserzeugungseinrichtung bzw. einen Wechselspannungsgenerator auf, welche dazu geeignet und bestimmt ist, eine Wechselspannung zu erzeugen, welche zum ionisieren der Luft verwendbar ist.

Bevorzugt setzt sich dabei dieser Wechselspannungsgenerator aus mehreren Komponenten zusammen, die besonders bevorzugt hintereinander geschaltet sind. Dies wird untenstehend unter Bezugnahme auf die Figuren erläutert.

Bevorzugt weist die Wechselspannung einen periodischen Verlauf auf. Besonders bevorzugt ist dieser Verlauf aus eine Gruppe von Verläufen ausgewählt, welche sinusförmige oder sinusähnliche Verläufe, rechtecksförmige Verläufe, sägezahnförmige Verläufe, dreiecksförmige Verläufe, Kombinationen und dergleichen enthält.

Besonders bevorzugt erzeugt der Wechselspannungsgenerator oder ein von dem Wechselspannungsgenerator gespeister Transformator eine periodisch verlaufende Wechselspannung. Bei dieser Wechselspannung kann es sich um eine Wechselspannung mit einem zeitlichen Sinusprofil, einem zeitlichen Dreiecksprofil, einem zeitlichen Rechtecksprofil oder dergleichen handeln.

Bevorzugt wird ein zeitlicher Verlauf erzeugt, bei dem die ausgegebene Spannung gegenüber einem sinusförmigen Verlauf über eine längere Zeitspanne hinweg oberhalb eines vorgegebenen Grenzwerts liegt. Ein Beispiel für einen solchen zeitlichen Verlauf ist ein rechteckförmiger zeitlicher Verlauf. Derartige Verläufe können insbesondere durch Halbleiterschaltungen erzeugt werden.

Bevorzugt ist diese periodische Wechselspannung (insbesondere bezogen auf die höchsten Spannungswerte) größer als 100V, bevorzugt größer als 200V, bevorzugt größer als 400kV, bevorzugt größer als 600V, bevorzugt größer als 1kV, bevorzugt größer als 2kV, bevorzugt größer als 4kV.

Bevorzugt ist diese periodische Wechselspannung (insbesondere bezogen auf die höchsten Spannungswerte) kleiner ist als 40kV, bevorzugt kleiner als 30kV, bevorzugt kleiner als 20kV, bevorzugt kleiner als 15kV.

Diese Spannungswerte sind für einen die Vorrichtung haltenden Nutzer nicht gefährlich, da nur sehr geringe Ströme fließen. Dennoch sind bevorzugt die spannungsführenden Bauteile der Vorrichtung elektrisch gegenüber dem Benutzer und insbesondere einer Hand des Benutzers, welche die Vorrichtung hält isoliert.

Bei einer weiteren bevorzugten Ausführungsform versorgt die Energiewandlereinrichtung die Ionisierungseinheit wenigstens mittelbar mit elektrischer Energie versorgt. Bei dieser Ausgestaltung ist daher eine Energiewandlereinheit vorgesehen, welche wenigstens einen Teil der Strömungsenergie der Druckluft in elektrische Energie und insbesondere in eine Wechselspannung umwandelt. Diese Wechselspannung und insbesondere eine aus dieser Wechselspannung gewonnene Spannung wird wiederum der Ionisierungseinheit zugeführt um auf diese Weise die Ionisierung der Luft zu ermöglichen.

Bei einer weiteren bevorzugten Ausführungsform weist der Wechselspannungsgenerator eine Wechselspannungswandlereinrichtung auf, welche eine Eingangsspannung (mit einem vorgegebenen im Wesentlichen konstanten Verlauf und bevorzugt eine Eingangswechselspannung mit einem ersten vorgegebenen zeitlichen Verlauf in eine Wechselsausgangsspannung mit einem zweiten vorgegebenenen Verlauf wandelt, wobei der zweite vorgegebene zeitliche Verlauf von dem ersten vorgegebenen zeitlichen Verlauf abweicht.

Bevorzugt unterscheiden sich der erste zeitliche Verlauf und der zweite zeitliche Verlauf in wenigstens einer charakteristischen Größe voneinander. Hierbei handelt es sich insbesondere um eine Größe, welche das zeitliche Verhalten der Spannung beschreibt, wie etwa ein dreiecksförmiges Profil oder ein rechteckförmiges Profil im Vergleich zu einem Sinusprofil.

Bei dieser Ausgestaltung bewirkt also die Wechselspanungswandlereinrichtung eine Änderung des zeitlichen Verlaufs der Wechselspannung. So kann beispielsweise Eingangswechselspannung, welche von der Generatoreinrichtung erzeugt wurde und welche einen Sinusförmigen Verlauf aufweist, in eine Ausgangswechselspannung mit einem dreiecksförmigen Verlauf oder einem rechtecksförmigen Verlauf umgewandelt werden. Es wäre auch denkbar, dass der zeitliche Verlauf der Ausgangsspannung eine zusammensetzung aus unterschiedlichen Verläufen aufweist, wie etwa zunächst einen Sinusförmigen Anstieg und dann ab einem bestimmten Grenzwert einen konstanten Verlauf.

Es wäre jedoch auch möglich, dass es sich bei der Eingangsspannung um eine Gleichspannung handelt und die Wechselspannungswandlereinrichtung aus dieser Gleichspannung eine Wechselspannung mit einem vorgegbenen zeitlichen Verlauf erzeugt.

Es wäre daher auch möglich, dass die Generatoreinrichtung eine Gleichspannung erzeugt.

Bei einer weiteren bevorzugten Ausführungsform weist die Wechselspannungswandlereinrichtung weist eine Erdung auf. So wäre es möglich, dass beispielsweise die Ausgangspannung über einen Erdungsausgang und einen Spannungsführungsausgang ausgegeben wird.

Bevorzugt ist die Wechselspannungseinrichtung über eine Druckanschlussleitung geerdet.

Bei einer weiteren bevorzugten Ausführungform weist die Wechselspannungswandlereinrichtung eine Vielzahl von Halbleiterelementen auf.

Bevorzugt weist die Wechselspannungswandlereinrichtung eine Vielzahl von Dioden auf. Diese können beispielsweise eine Gleichrichtung der Eingangsspannung bewirken.

Weiterhin weist bevorzugt die Wechselspannungswandlereinrichtung wenigstens eine Glättungseinrichtung zum Glätten einer Eingangsspannung auf. Diese Glättungseinrichtung weist dabei bevorzugt wenigstens einen Kondensator auf.

Bei einer weiteren bevorzugten Ausführungsform weist die Wechselspannungswandlereinrichtung wenigsten einen Transistor und bevorzugt wenigstens zwei Transistoren auf. Bevorzugt dienen diese Transistoren zum Erzeugen einer Wechselspannung mit einem zeitlichen Profil, welches von einem Sinusprofil abweicht.

Bei einer weiteren bevorzugten Ausführungsform weist die Wechselspannungswandlereinrichtung eine Gleichrichtereinheit auf. Diese dient insbesondere zum Gleichrichten der Eingangsspannung.

Die Anmelderin hat herausgefunden, dass eine Sinusspannung zur Ionisierung eher weniger geeignet ist, da nur bestimmte Anteile des zeitlichen Verlaufs eine Ionisierung bewirken. Die hier beschriebenen Dreiecksspannungen oder Rechtecksspannungen haben sich als besser geeignet zum Ionisieren der Luft mit unterschiedlichen Polaritäten erwiesen.

Bevorzugt ist ein Ionisierungselement der Ionisierungseinheit derart ausgebildet, dass Druckluft an zwei Elektrodenbereichen oder zwischen zwei Elektrodenbereichen vorbeiströmt.

Bevorzugt ist ein Ionisierungselement der Ionisierungseinheit derart ausgebildet, dass der Strömungsabschnitt, entlang dessen die Druckluft während der Ionisierung strömt kürzer ist als 20cm, bevorzugt kürzer als 15cm, bevorzugt kürzer als 10cm, bevorzugt kürzer als 8cm, bevorzugt kürzer als 6cm, bevorzugt kürzer als 4cm, bevorzugt kürzer als 3cm und besonders bevorzugt kürzer als 2cm.

Bevorzugt ist ein Ionisierungselement der Ionisierungseinheit derart ausgebildet, dass der Strömungsabschnitt, entlang dessen die Druckluft während der Ionisierung strömt länger ist als 0,05cm, bevorzugt länger als 0,1cm, bevorzugt länger als 0,2cm und besonders bevorzugt länger als 0,4cm.

Bei einer weiteren vorteilhaften Ausführungsform wird und/oder ist die Wechselspannungswandlereinrichtung (insbesondere unmittelbar) von der Energiewandlereinrichtung gespeist. Bei einer weiteren bevorzugten Ausführungsform wird oder ist ein Ausgangssignal und/oder eine Ausgangsspannung der Energiewandlereinrichtung wenigstens mittelbar der Wechselspannungswandlereinrichtung als Eingangssignal zugeführt.

Bei einer weiteren bevorzugten Ausführungsform weist die Wechselspannungswandlereinrichtung eine Transformatoreinrichtung auf, welche eine von einer Spannungserzeugseinheit der Wechselspannungswandlereinrichtung erzeugte Zwischenspannung in eine Ausgangsspannung der Wechselspannungswandlereinrichtung transformiert und insbesondere hochtransformiert.

Bei einer weiteren vorteilhaften Ausführungsform weist die Vorrichtung eine Ladungserzeugungseinrichtung auf, welche dazu geeignet und bestimmt ist, die zu beaufschlagende Oberfläche elektrisch aufzuladen und insbesondere diese mit einem bevorzugt konstanten Potential aufzuladen. Bei dieser Einrichtung kann es sich etwa um ein textiles Element wie etwa ein Tuch handeln, welches die Ladung erzeugt, wenn es über die Oberfläche des Fahrzeugs bewegt wird. Dabei ist nicht ohne weiteres vorhersehbar, mit welcher Polarität die Oberfläche aufgeladen wird.

Die Beaufschlagung sowohl mit positiv als auch negativ ionisierter Luft bewirkt jedoch bevorzugt einer Entladung der Oberfläche.

Bei einer weiteren bevorzugten Ausführungsform erzeugt der Wechselspannungsgenerator eine periodisch verlaufende Wechselspannung und diese periodische Wechselspannung weist eine Frequenz aufweist, welche höher ist als 100Hz, bevorzugt höher als 200Hz, bevorzugt höher als 400Hz, bevorzugt höher als 800Hz und bevorzugt höher als 1000Hz und besonders bevorzugt höher als 2000Hz.

Bevorzugt weist diese Wechselspannung einer Frequenz auf, die geringer ist als 100kHz, bevorzugt als 50kHz, bevorzugt niedriger als 40kHz, bevorzugt niedriger als 30kHz, bevorzugt niedriger als 20kHz.

Vorteilhaft weist die Vorrichtung einen Griffabschnitt auf, welcher zum Halten der Vorrichtung dient, sowie einen Ausgabeabschnitt, der zum Ausgeben der ionisierten Luft dient, wobei besonders bevorzugt die Energiewandlereinrichtung wenigstens abschnittsweise in den Griffabschnitt integriert ist. Bevorzugt ist eine Generatoreinrichtung der Energiewandlereinrichtung in dem Griffabschnitt angeordnet.

Besonders bevorzugt erzeugt die Energiewandlereinrichtung unmittelbar die Wechselspannung zum Ionisieren der Luft. Bevorzugt ist daher die Energiewandlereinrichtung geeignet, eine Wechselspannung zu erzeugen.

Auf diese Weise kann eine bequemere Anordnung der Vorrichtung erreicht werden, da vergleichsweise schwere Komponenten in den Griffbereich integriert sind. Weiterhin kann auch der Luftstrom der zugeführten Druckluft in besonders günstiger Weise genutzt werden, da sich die Energiewandlung sehr nahe an der Zuführung der Druckluft befindet.

Bevorzugt erstrecken sich der Griffabschnitt und der Ausgabeabschnitt in unterschiedliche Richtungen. Dabei verlaufen diese Richtungen besonders bevorzugt derart, dass zwischen den Richtungen ein Winkel gebildet wird, der größer ist als 50°, bevorzugt größer als 60°, bevorzugt größer als 70° und bevorzug größer als 80°. Bevorzugt ist dieser Winkel kleiner als 150°, bevorzugt kleiner als 130°, bevorzugt kleiner als 120°, bevorzugt kleiner als 110° und besonders bevorzugt kleiner als 100°.

Bei einer vorteilhaften Ausführungsform weist die Energiewandlereinrichtung eine Generatoreinrichtung auf, welche eine Drehbewegung eines Rotors gegenüber einem Stator in elektrische Energie wandelt, wobei die Generatoreinrichtung besonders bevorzugt als Außenläufermaschine ausgebildet ist. Bei einer besonders bevorzugten Ausführungsform ist die Energiewandlereinrichtung und/oder die Generatoreinrichtung in den Griffabschnitt integriert.

Auch durch die Ausbildung der Generatoreinrichtung als Außenläufermaschine kann die Ergonomik der Vorrichtung sowie die Bequemlichkeit der Vorrichtung verbessert werden, da durch die Ausbildung der Generatoreinrichtung als Außenläufermaschine diese kleiner gestaltet werden kann und auch die Luftströmungskanäle im Inneren des Gehäuses platzsparender angeordnet werden können.

Dies bedeutet, dass bevorzugt der Stator der Generatoreinrichtung innenliegend angeordnet ist und der sich gegenüber dem Stator drehende Rotor außen. Ein Außenläufer ist eine Bauform einer rotierenden elektrischen Maschine, bei der sich der ruhende Teil (Stator) der Maschine in ihrem Innern befindet und vom bewegten Teil (Rotor, "Läufer") umschlossen ist. Die Maschine kann je nach Betriebspunkt und Ausführung als elektrischer Generator oder als Elektromotor verwendet werden.

Der Vorteil in der Ausbildung der Generatoreinrichtung als Außenläufermaschine besteht darin, dass der Luftstrom in günstigerer Weise genutzt werden kann, um die Drehbewegung des Rotors zu erzeugen. Auch kann auf diese Weise die Generatoreinrichtung kleiner und damit leichter ausgeführt sein.

Eine weitere erfindungsgemäße Vorrichtung zum Beaufschlagen von Oberflächen und insbesondere von Kraftfahrzeugoberflächen mit ionisierter Luft weist einen Zuführanschluss auf, um der Vorrichtung Druckluft zuzuführen. Weiterhin weist die Vorrichtung eine Ionisierungseinheit auf, welche dazu geeignet und bestimmt ist, wenigstens einen Anteil der über den Zuführanschluss zugeführten Luft zu ionisieren. Daneben weist die Vorrichtung eine Ausgabeeinrichtung auf, welche ionisierte Luft ausgibt und insbesondere an eine Oberfläche ausgibt.

Weiterhin weist die Ionisierungseinheit eine Energiewandlereinrichtung auf, welche eine Bewegungsenergie des Luftstroms wenigstens teilweise in eine elektrische Energie umwandelt. Erfindungsgemäß weist die Energiewandlereinrichtung eine Generatoreinrichtung auf, welche eine Drehbewegung eines Rotors gegenüber einem Stator in elektrische Energie wandelt, wobei bevorzugt die Energiewandlereinrichtung als Außenläufermaschine ausgebildet ist. Bevorzugt erzeugt die Energiewandlereinrichtung eine Wechselspannung.

Bei einer vorteilhaften Ausführungsform weist der Rotor der Generatoreinrichtung an seinem Außenumfang Luftaufnahmeeinrichtungen wie etwa Luftschaufeln auf, welche von der vorbeiströmenden Luft bewegt werden können. Bevorzugt verläuft wenigstens abschnittsweise um die Generatoreinrichtung ein Kanal, durch welchen die zugeführte Druckluft strömt.

Bei einer weiteren bevorzugten Ausführungsform weist eine Drehachse des Rotors der Generatoreinrichtung eine Komponente in einer Richtung auf, die sich in der Austrittsrichtung der aus der Vorrichtung austretenden Luft erstreckt.

Bevorzugt spannen die Austrittsrichtung der aus der Vorrichtung austretenden (ionisierten) Luft und die Zuführrichtung eine Ebene auf und die Drehachse des Rotors erstreckt sich ebenfalls in dieser Ebene oder in einer hierzu parallelen Ebene.

Bevorzugt erstreckt sich die Drehachse des Rotors gegenüber einer Hauptströmungsrichtung der aus der Vorrichtungen austretenden Luft in einem Winkel, der geringer ist als 40°, bevorzugt geringer als 30°, bevorzugt geringer als 25°, bevorzugt geringer als 20°, bevorzugt geringer als 15° und bevorzugt geringer als 10°.

Bei einer weiteren vorteilhaften Ausführungsform ist die Generatoreinrichtung wenigstens teilweise und besonders bevorzugt vollständig in den Griffabschnitt der Vorrichtung integriert.

Bei einer weiteren vorteilhaften Ausführungsform weist die Vorrichtung wenigstens zwei Gehäuseeinheiten auf, wobei die Energiewandlereinrichtung in der ersten Gehäuseeinheit angeordnet ist und diese erste Gehäuseeinheit wenigstens teilweise innerhalb der zweiten Gehäuseeinheit angeordnet ist.

Es wird darauf hingewiesen, dass diese Variante auch unabhängig von der oben beschriebenen Ausgestaltung Vorteile erzielt, insbesondere im Hinblick auf eine elektrische Isolierung der Vorrichtung. Allerdings können sich auch spezifische Vorteile aus der Kombination beider Ausführungsformen ergeben, da auf diese Weise eine bessere elektrische Isolierung gerade auch gegenüber der Generatoreinrichtung, die sich bevorzugt in dem Griffabschnitt befindet, erreicht wird.

Eine erfindungsgemäße Vorrichtung zum Beaufschlagen von Oberflächen und insbesondere von Kraftfahrzeugoberflächen mit ionisierter Luft weist einen Zuführanschluss auf, um der Vorrichtung Druckluft zuzuführen. Weiterhin weist die Vorrichtung eine Ionisierungseinheit auf, welche dazu geeignet und bestimmt ist, wenigstens einen Anteil der über den Zuführanschluss zugeführten Luft zu ionisieren. Daneben weist die Vorrichtung eine Ausgabeeinrichtung auf, welche ionisierte Luft aus-gibt und insbesondere an eine Oberfläche ausgibt. Weiterhin weist die Ionisierungseinheit eine Energiewandlereinrichtung auf, welche eine Bewegungsenergie des Luftstroms wenigstens teilweise in eine elektrische Energie umwandelt.

Erfindungsgemäß weist die Vorrichtung wenigstens zwei Gehäuseeinheiten auf, wobei die Energiewandlereinrichtung in der ersten Gehäuseeinheit angeordnet ist und diese erste Gehäuseeinheit wenigstens teilweise innerhalb der zweiten Gehäuseeinheit angeordnet ist. Insbesondere erzeugt die Energiewandlereinrichtung eine Wechselspannung.

Bei einer weiteren bevorzugten Ausführungsform ist die zweite Gehäuseeinheit als umgebendes Gehäuse ausgebildet. Bevorzugt ist der zweite Gehäuseteil aus einem elektrisch isolierenden Material und insbesondere aus Kunststoff ausgebildet. Besonders bevorzugt ist die gesamte Außenoberfläche des Gehäuses aus einem Kunststoff ausgebildet. Auf diese Weise kann verhindert werden, dass ein Nutzer mit der zur Ionisierung verwendeten Spannung in Berührung kommt.

Bevorzugt ist der zweite Gehäuseteil wenigstens in dem Griffabschnitt der Vorrichtung ausgebildet. Es wäre jedoch auch denkbar, dass der zweite Gehäuseteil den ersten Gehäuseteil im Wesentlichen vollständig umgibt.

Bei einer weiteren bevorzugten Ausführungsform ist der zweite Gehäuseteil mehrteilig und/oder wenigstens zweiteilig ausgebildet. Dabei ist es möglich, dass diese beiden Gehäuseteile mittels eines Clipmechanismus aneinander befestigt werden.

Bei einer weiteren bevorzugten Ausführungsform weist die zweite Gehäuseeinheit wenigstens zwei miteinander verbindbare Gehäuseteile auf. Bevorzugt sind diese beiden Gehäuseteile (zumindest auch) durch einen Druckluftanschluss aneinander gehalten.

Bei einer weiteren vorteilhaften Ausführungsform weist die Vorrichtung eine Sensoreinrichtung zur Erfassung eines Luftdrucks oder eines Luftstroms auf. Hierbei kann es sich beispielsweise um einen Durchflussmesser oder um einen Druckmesser handeln. Es wäre jedoch auch möglich, dass es sich bei der Sensoreinrichtung um eine Strom- oder Spannungsmesseinrichtung handelt, welche eine von der Energiewandlereinrichtung erzeugte Spannung oder einen Strom misst, da diese Spannung bzw. dieser Strom ein Maß für den Druck der Druckluft bzw. dessen Strömungsgeschwindigkeit ist.

Bei einer weiteren bevorzugten Ausführungsform weist die Vorrichtung eine Warneinrichtung auf, die bei einer Abweichung eines Ist-Drucks von einem Solldruck, insbesondere bei einem zu hohen oder zu niedrigen Luftdruck ein Warnsignal ausgibt. Insbesondere handelt es sich bei der Warneinrichtung um eine optische bzw. optisch wahrnehmbare Warneinrichtung. Diese kann dabei beispielsweise in einem Übergangsbereich zwischen dem Griffabschnitt und dem Ausgabeabschnitt angeordnet sein, um so stets von einem Benutzer gut wahrnehmbar zu sein.

Dabei ist es möglich, dass diese Warneinrichtung keine eigene Stromquelle aufweist, sondern von der Energiewandlereinrichtung mit Energie versorgt wird.

Es wird daher vorgeschlagen, dass bevorzugt der Luftstrom der Luft selbst genutzt wird, um die Ionisierung zu erreichen. Insbesondere ist daher eine Energiewandlereinheit vorgesehen, welche eine Umwandlung von Bewegungsenergie des Luftstroms in elektrische Energie ermöglicht.

Anstelle von Luft könnte auch ein anderes gasförmiges Medium verwendet werden, beispielsweise sterilisierte Luft oder ein Sterilgas.

Bevorzugt ist die Ausgabeeinrichtung, bei der es sich insbesondere um eine Luftausgabeeinrichtung handelt, wiederum an der Ionisierungseinheit angeordnet. So kann die Ausgabeeinrichtung an der Ionisierungseinheit angeschraubt sein. Die Luftausgabeeinrichtung kann dabei bevorzugt auch dazu geeignet und bestimmt sein ein elektrisches Feld zur Ionisierung zu erzeugen.

Bei einer bevorzugten Ausführungsform weist die Vorrichtung eine Leuchteinrichtung auf, welche ein optisch wahrnehmbares Signal ausgibt und welche insbesondere aufleuchtet, wenn die Energiewandlereinrichtung von dem Luftstrom mit Energie versorgt wird.

Bei einer bevorzugten Ausführungsform kann die Vorrichtung eine Luftfiltereinheit aufweisen, um die zugeführte Luft zu filtern. Weiterhin kann die Vorrichtung eine Trocknungseinheit zum Trocknen von durchströmender Luft aufweisen. Es wäre jedoch auch möglich, dass die getrocknete und/oder sterilisierte oder gereinigte Luft bereits über den Zuführanschluss zugeführt wird.

Bei einer weiteren vorteilhaften Ausführungsform weist die Energiewandlereinrichtung ein Piezoelement und/oder eine Dynamoeinrichtung auf. Auf diese Weise kann erreicht werden, dass der Luftstrom beispielsweise mittelbar ein Piezoelement bewegt und so Spannungen erzeugt, oder aber dass der Luftstrom etwa eine Dynamoeinheit antreibt und diese wiederum eine Wechselspannung und insbesondere eine Wechselspannung erzeugt.

Bei den genannten Ausführungsformen erlaubt daher die Vorrichtung allein mit dem Luftstrom versorgt zu werden, wobei der besagte Luftstrom auch die zum Ionisieren nötige Energie zur Verfügung stellt.

Bei einer weiteren vorteilhaften Ausführungsform weist die Energiewandeleinrichtung ein drehbares Turbinenelement auf, welches insbesondere durch den besagten Luftstrom in eine Drehung versetzbar ist. So kann es sich beispielsweise um eine Art Schaufelrad handeln, welches durch den Luftstrom in Drehung versetzt wird. Diese Drehung wiederum bewirkt die Erzeugung elektrischer Energie durch die Energiewandlereinrichtung. Wie oben erwähnt, ist dabei dieses Turbinenrad bevorzugt Bestandteil einer Außenläufermaschine.

Bei einer weiteren vorteilhaften Ausführungsform schließt sich an das Turbinenelement ein Luftkanal an, der die Luft letztlich zu der Ausgabeeinrichtung fördert.

Bei einer weiteren vorteilhaften Ausführungsform weist die Ionisierungseinheit eine Hochspannungserzeugungseinheit auf. Bevorzugt erzeugt diese Hochspannungserzeugungseinheit eine Wechselspannung. So kann es sich beispielsweise bei der Hochspannungserzeugungseinheit um einen Transformator handeln, der die von der Energiewandlereinrichtung zugeführte Wechselspannung in eine Hochspannung transformiert. Bevorzugt ist dabei diese Hochspannungserzeugungseinheit außerhalb des Griffabschnitts angeordnet und besonderes bevorzugt in dem Ausgabeabschnitt. Bevorzugt ist diese Hochspannungserzeugungseinheit dazu geeignet und bestimmt, eine Wechselspannung zu erzeugen.

Diese Hochspannung kann anschließend an einen Bereich der Ausgabeeinrichtung angelegt werden. So kann beispielsweise eine Elektrode bzw. elektrische Leitung von der Spannungserzeugungseinheit zu einer Düsenkappe führen und die zweite Leitung etwa zu einer bezüglich dieser Düsenkappe zentral gelegenen Nadel, sodass zwischen diesen beiden Elementen ein elektrisches Feld aufgebaut werden kann, welches letztlich die Ionisierung der Luft bewirkt.

Daneben wäre es jedoch auch möglich, dass die Vorrichtung eine Gleichrichtereinheit aufweist, welche dazu geeignet und bestimmt ist, die von der Hochspannungserzeugungseinheit erzeugte Wechselspannung in eine Gleichspannung umzuwandeln.

Bei einer weiteren bevorzugten Ausführungsform weist die Vorrichtung wenigstens einen Kondensator und/oder wenigstens eine Diode auf. Diese Elemente können beispielsweise zum Gleichrichten oder auch zum Glätten der von der Hochspannungserzeugungseinrichtung ausgegebenen Spannung dienen.

Bei einer weiteren vorteilhaften Ausführungsform weist die Hochspannungserzeugungseinheit wie oben erwähnt eine Transformatoreinheit auf.

Bei einer weiteren vorteilhaften Ausführungsform ist die Hochspannungserzeugungseinheit elektrisch mit der Energiewandlereinheit versorgbar und/oder verbunden.

Bei einer weiteren vorteilhaften Ausführungsform weist die Ausgabeeinrichtung ein Düsenelement auf, über welches ein Luftstrom insbesondere an die zu beaufschlagende Oberfläche aufbringbar ist.

Dabei kann bevorzugt, wie oben erwähnt, dieses Düsenelement eine der beiden elektrischen Elektroden sein, welche elektrisch mit der Wechselspannungs- oder Hochspannungserzeugungseinheit verbunden sind. Bei einer weiteren vorteilhaften Ausführungsform weist die Vorrichtung eine Strömungsverteilungseinrichtung auf, welche einen strömenden Luftstrom auf bevorzugt mehrere Öffnungen aufteilt. Diese mehreren Öffnungen können dabei in Umfangsrichtung um eine zentral angeordnete Elektrode angeordnet sein. Besonders bevorzugt können dabei diese Öffnungen gleichmäßig und/oder unregelmäßig verteilt sein.

Bei einer weiteren bevorzugten Ausführungsform weist die Vorrichtung eine Erdungseinrichtung auf, um die Vorrichtung in einem Betrieb elektrisch zu erden. Besonders bevorzugt steht eine der beiden Elektroden mit dieser Erdungseinrichtung in elektrisch leitender Verbindung. Bevorzugt ist daher wenigstens eine der beiden Elektroden elektrisch geerdet.

Bevorzugt sind auch elektrische Komponenten der Wechselspannungswandlereinrichtung über die Erdungseinrichtung geerdet.

Bei einer weiteren bevorzugten Ausführungsform weist die Ausgabeeinrichtung ein elektrisch leitendes Anschlussmittel auf, um die Ausgabeeinrichtung mit einer Erdungsleitung zu verbinden. So kann beispielsweise ein Befestigungsmittel, an dem ein Druckschlauch an der Ausgabeeinrichtung oder an der Vorrichtung befestigt wird, auch dazu dienen, eine elektrisch leitende Verbindung zum Zweck einer Erdung herzustellen.

Bei einer weiteren bevorzugten Ausführungsform ist wenigstens ein Parameter der für die von der Ionisierungseinheit zur Ionisierung verwendete Spannung charakteristisch ist veränderbar ist. Auf diese Weise kann ein Benutzer die Spannung anpassen etwa wenn ihm bekannt ist, wie die Oberfläche geladen ist oder etwa in Abhängigkeit von den zu behandelnden Oberflächen.

Bevorzugt ist dieser Parameter aus einer Gruppe von Parametern ausgewählt ist, welche eine Frequenz der Spannung, eine maximale positive Spannung, eine maximale negative Spannung, einen zeitlichen Verlauf der Spannung eine Symmetrie der Spannung bezüglich eines vorgegebenen Potenzials und dergleichen enthält.

Bei einer weiteren vorteilhaften Ausführungsform ist über den Zuführanschluss Luft unter einem Druck zuführbar, der größer ist als 0,1 bar, bevorzugt größer als 0,5 bar, bevorzugt größer als 1 bar, bevorzugt größer als 2 bar.

Weiterhin ist bevorzugt über den Zufuhranschluss Luft unter einem Druck zuführbar, der kleiner ist als 15 bar, bevorzugt kleiner als 12 bar, bevorzugt kleiner als 10 bar, bevorzugt kleiner als 9 bar und besonders bevorzugt kleiner als 8 bar.

Diese Drücke haben sich als besonders vorteilhaft herausgestellt, um auch bei einer nachfolgenden Ionisierung noch eine ausreichende Beaufschlagung der jeweiligen Oberflächen mit Luft zu erreichen.

Bei einer weiteren vorteilhaften Ausführungsform weist die Vorrichtung eine Druckregelungseinrichtung auf, welche einen Druck der zu der Energiewandeleinrichtung gelangenden Luft bzw. des zu der Energiewandeleinrichtung gelangenden Luftstroms bewerkstelligt. Bei einer bevorzugten Ausführungsform ist diese Druckregeleinrichtung an dem Luftanschluss angeordnet. Besonders bevorzugt ist die Druckregeleinrichtung in Richtung des Luftstroms stromaufwärts bezüglich des Anschlusses angeordnet. Auf diese Weise ist eine besonders stabile Ausführung der Druckregeleinrichtung möglich.

Mit Hilfe der Druckregeleinrichtung kann bereits vor dem Erreichen der Energiewandeleinrichtung der jeweilige gewünschte Druck eingestellt werden.

Bei einer weiteren vorteilhaften Ausführungsform handelt es sich bei der Vorrichtung um eine mit der Hand bzw. von Hand tragbare Vorrichtung. So kann beispielsweise die Einrichtung als Sprühpistole ausgebildet sein, die von einem Benutzer gehalten werden kann, um so die Oberfläche der Kraftfahrzeuge mit ionisierter Luft zu beaufschlagen. Bei einer weiteren bevorzugten Ausführungsform weist der Zuführanschluss ein Metallgewinde auf, über welches eine (Luft)Druckquelle angeschlossen werden kann.

Bei einer weiteren bevorzugten Ausführungsform weist die Vorrichtung eine Beleuchtungseinrichtung zur Beleuchtung der zu bearbeitenden Oberfläche und insbesondere Kraftfahrzeugoberfläche auf. Dabei kann diese Beleuchtungseinrichtung beispielsweise am Kopf der Vorrichtung angeordnet sein und auf die zu beaufschlagende Oberfläche ausgerichtet sein. Bei einer bevorzugten Ausführungsform weist die Beleuchtungseinrichtung wenigstens eine und bevorzugt mehrere Lichtquellen, insbesondere Leuchtdioden auf. Dabei können diese Lichtquellen um eine Austrittsöffnung herum aus welcher die ionisierte Luft austritt, angeordnet sein. Auf diese Weise kann die lonisierfläche besser erkennbar sein.

Bei einer bevorzugten Ausführungsform weist die Beleuchtungseinrichtung wenigstens eine Weisslicht-Diode auf

Bei einer bevorzugten Ausführungsform gibt die Beleuchtungseinrichtung das Licht in Form eines Lichtkegels aus, der dazu geeignet ist, eine Fläche zu beleuchten, die zwischen 1 cm² und 1000 cm². Bevorzugt ist dabei die Größe dieses Lichtkegels veränderbar.

Bei einer bevorzugten Ausführungsform weist die Beleuchtungseinrichtung eine LED und/oder einen Laser und/oder eine Laserdiode auf. Bei einer weiteren bevorzugten Ausführungsform weist die Beleuchtungseinrichtung ein optisch refraktives Element und insbesondere eine Linseneinrichtung auf.

Bei einer bevorzugten Ausführungsform ist eine Brennweite dieser Linseneinrichtung derart gewählt, dass sie das aus der Beleuchtungseinrichtung austretende Licht in etwa der gleichen Weise aufweitet wie der ausgegebene Luftstrom (der durch die Düse ausgegeben wird).

Auf diese Weise ist es möglich, dass ein Lichtfleck unabhängig von seinem Abstand in etwa so groß ist wie der "Sprühkegel" der ausgegebenen Luft.

Bei einer weiteren bevorzugten Ausführungsform ist die Beleuchtungseinrichtung derart angeordnet und/oder ausgerichtet, dass sie auf dem zu behandelnden Werkstück einen Lichtfleck erzeugt, der zumindest teilweise deckungsgleich mit demjenigen Bereich ist, auf den die ionisierte Luft auftritt. Bevorzugt ist der von der Beleuchtungseinrichtung auf die zu bearbeitende Oberfläche ausgebbare Lichtfleck kleiner als der von der Vorrichtung erreichte Bereich der ionisierten Luft. Auf diese Weise kann sichergestellt werden, dass sich ein Benutzer, der die Vorrichtung verwendet und sich an dem Lichtfleck orientiert, jedenfalls alle gewünschten Oberflächenareale ionisiert.

Bevorzugt ist die Beleuchtungseinrichtung ist gleichzeitig mit dem Sprühvorgang auslösbar und aktivierbar. So kann etwa ein Drücktaster vorgesehen sein, der zeitglich oder zeitlich gering versetzt.

Bevorzugt wird die Beleuchtungseinrichtung mit einer von dem Generator aus dem Luftstrom erzeugten elektrischen Energie gespeist. Es wäre jedoch auch möglich, dass die Vorrichtung eine aufladbare Energiespeichereinrichtung wie eine Batterie oder einen Kondensator aufweist, welche bevorzugt von der von dem Generator erzeugten Energie gespeist wird.

Bei einer weiteren bevorzugten Ausführungsform ist die Beleuchtungseinrichtung dazu geeignet und bestimmt einen Lichtkegel und/oder einen Lichtfleck auf der zu bearbeitenden Oberfläche auszubilden, der einen von kreisförmigen Querschnitt abweichenden Querschnitt aufweist. Insbesondere handelt es sich hierbei um einen elliptischen Querschnitt.

Besonders bevorzugt weist die Beleuchtungseinrichtung wenigstens ein optisches Element auf, welches geeignet ist einen Lichtkegel und/oder eine Beleuchtung der zu bearbeitenden Oberfläche mit einem elliptischen Querschnitt zu erreichen. Besonders bevorzugt handelt es sich bei dem optischen Element um eine Zylinderlinse.

Die Erzeugung eins elliptischen Lichtflecks ermöglich eine genauere Anpassung an das Profil der von der Vorrichtung ausgegebenen Luftstroms auf die Oberfläche.

Besonders bevorzugt ist eine Elliptizität der von der Beleuchtungseinrichtung ausgegebenen Beleuchtung veränderbar. Dies kann beispielsweise durch eine Einstellung eines Abstands zwischen der oder den Lichtquellen und einem optischen Element erfolgen.

Bei einer weiteren bevorzugten Ausführungsform weist die Vorrichtung eine Abstandserfassungseinrichtung auf, welche einen Abstand der Vorrichtung zu einer zu beaufschlagenden Wand erfasst und insbesondere berührungslos erfasst. Dabei kann es sich beispielsweise um eine Infrarot Abstandserfassungseinrichtung handeln.

Bei einer weiteren bevorzugten Ausführungsform weist die Vorrichtung eine Steuerungseinrichtung auf, welche die Beleuchtungseinrichtung in Reaktion auf einen von der Abstandserfassungseinrichtung ermittelten Abstand steuert. So ist es beispielsweise möglich, dass die Beleuchtung abgeschaltet wird, wenn ein Abstand zwischen der Vorrichtung und der zu beaufschlagenden Oberfläche zu groß ist bzw. einen vorgegebenen Schwellenwert übersteigt.

Bei einer weiteren bevorzugten Ausführungsform wird auch die Abstandserfassungseinrichtung mit elektrischer Energie gespeist, die aus dem Luftstrom gewonnen wird.

Bei einer weiteren bevorzugten Ausführungsform weist die Vorrichtung eine Zeiterfassungseinrichtung auf. Dabei ist es möglich, dass die Zeiterfassungseinrichtung zeitliche Größen erfasst, wie etwa die Uhrzeit und/oder Zeitdauer eines Arbeitsvorgangs. Daneben können mit der Zeiterfassungseinrichtung Betriebsstunden erfasst und/oder gezählt werden.

Bei einer weiteren bevorzugten Ausführungsform weist die Vorrichtung eine Speichereinrichtung auf, in der Zeitwerte und/oder Zeitdauern speicherbar sind. Auf diese Weise sind Arbeitsvorgänge protokollierbar. Weiterhin weist bevorzugt die Vorrichtung eine Kommunikationsschnittstelle auf, im etwa mit einem Computer kommunizieren zu können. Diese Kommunikationsschnittstelle kann dabei kabelgebunden oder drahtlos arbeiten.

Bei einer weiteren vorteilhaften Ausführungsform ragen das Piezoelement oder ein das Piezoelement stützender oder tragender Träger wenigstens abschnittsweise in eine Strömung der Luft. Auf diese Weise kann das Piezoelement direkt durch den Luftstrom zu Bewegungen angeregt werden und so eine Spannung erzeugen.

Bei einer weiteren vorteilhaften Ausführungsform ermöglicht der Zuführanschluss einen Strömungsquerschnitt der in die Vorrichtung eintretenden Luft, der größer ist als 0.05 mm, bevorzugt größer als 0.1 mm und besonders bevorzugt größer als 0,15 mm und besonders bevorzugt größer als 0,2 mm.

Bevorzugt ermöglicht der Zuführanschluss einen Strömungsquerschnitt der in die Vorrichtung eintretenden Luft, der kleiner ist als 20 mm, bevorzugt kleiner als 18 mm und bevorzugt kleiner als 15 mm und besonders bevorzugt kleiner als 10 mm.

Bei einer weiteren vorteilhaften Ausführungsform weist die Vorrichtung eine Ventileinrichtung auf, um den von dem Zuführanschluss zu der Ausgabeeinrichtung gelangenden Luftstrom zu steuern. Bevorzugt ist diese Ventileinrichtung in der Strömungsrichtung der Luft stromaufwärts bezüglich der Ionisierungseinheit angeordnet. Bevorzugt ist diese Ventileinrichtung zumindest mittelbar manuell betätigbar.

Bevorzugt ist dabei diese Ventileinrichtung derart ausgeführt, dass sie etwa von einem Schaltelement, wie etwa einem schwenkbaren Griff der Schalter oder dgl. betätigt werden kann. besonders bevorzugt ist dabei eine Rückstelleinrichtung und/oder eine Federeinrichtung vorgesehen, welche diese Ventileinrichtung in einen geschlossenen Zustand drängt, sodass ohne Betätigung der Vorrichtung die Ventileinrichtung einen Luftstrom blockiert.

Bei einer weiteren vorteilhaften Ausführungsform weist die Vorrichtung ein Auslöseelement auf, um diese Ventileinrichtung zu betätigen. So kann dieses Auslöseelement derart gestaltet sein, dass auch über das Auslöseelement ein Luftdruck der zu der Ausgabeeinrichtung gelangenden ionisierten Luft gesteuert werden kann.

Bei einer weiteren vorteilhaften Ausführungsform weist die Vorrichtung ein Gehäuse auf, in dem wenigstens abschnittsweise eine Luftleitung angeordnet ist. Diese Luftleitung kann dabei wenigstens abschnittsweise von dem Zuführanschluss zu der Ausgabeeinrichtung führen, wobei zwischen dem Zuführanschluss und dem Ausgabeelement bzw. der Ausgabeeinrichtung noch die Energiewandlereinheit und/oder das Turbinenrad angeordnet ist.

Bei einer weiteren vorteilhaften Ausführungsform weist die Vorrichtung eine Leitung zum Leiten der Luft auf und diese Leitung ändert eine Strömungsrichtung der Luft wenigstens einmal. So kann beispielsweise eine Leitungseinrichtung die Strömungsrichtung der Luft um einen vorgegebenen Winkel insbesondere einen Winkel zwischen 10° und 170° , etwa von 90° ändern.

Bei einer weiteren bevorzugten Ausführungsform ist die Vorrichtung dazu geeignet und bestimmt, eine statisch geladene Oberfläche durch die Beaufschlagung wenigstens teilweise zu entladen und/oder die Ladung zu eliminieren.

Bei einer weiteren bevorzugten Ausführungsform weist die Vorrichtung wenigstens ein mit einer Spitze ausgebildetes lonisierelement und bevorzugt wenigstens zwei mit einer Spitze ausgebildete lonisierelemente auf, welche an diesen lonisierelementen vorbeiströmende Luft ionisieren.

Die vorliegende Erfindung ist weiterhin auf ein Verfahren zum Bearbeiten und insbesondere zum Lackieren von Oberflächen von Kraftfahrzeugen gerichtet, wobei eine Kraftfahrzeugoberfläche mit ionisierter Luft beaufschlagt wird.

Erfindungsgemäß wird die ionisierte Luft von einer Vorrichtung der oben beschriebenen Art erzeugt. Besonders bevorzugt erzeugt dabei eine strömende Luft eine Spannung, die wiederum zum Ionisieren der Luft verwendet wird.

Die vorliegende Erfindung ist weiterhin auf ein Verfahren zum Sterilisieren und/oder Reinigen von Oberflächen gerichtet, wobei eine zu sterilisierende oder zu reinigende Oberfläche mit einem gasförmigen Medium und insbesondere mit Luft beaufschlagt wird.

Erfindungsgemäß wird die ionisierte Luft mittels einer Vorrichtung der oben beschriebenen Art erzeugt.

Bei einer bevorzugten Ausführungsform wird der Vorrichtung getrocknete Luft und/oder gereinigte Luft zugeführt. Besonders bevorzugt wird wie oben erwähnt Luft unter Drücken von wenigstens 0,5 bar, bevorzugt von wenigstens 1 bar und besonders bevorzugt von wenigstens 2 bar zugeführt. Besonders bevorzugt wird Luft unter einem Druck zugeführt, der kleiner ist als 15 bar, bevorzugt kleiner als 12 bar, bevorzugt kleiner als 10 bar und besonders bevorzugt kleiner als 8 bar.

Bei einem weiteren bevorzugten Verfahren wird die Luft durch eine Ausgabeeinrichtung, welche die Luft auch ausgibt, ionisiert. Vorteilhaft wird eine erzeugte Wechselspannung umgewandelt und insbesondere transformiert. Bei einem weiteren bevorzugten Verfahren wird die besagte Vorrichtung mit der Hand gehalten. Besonders bevorzugt wird die Luft unter einem Winkel zwischen 5° und 50° bezogen auf die Oberfläche bzw. die Ebene der Oberfläche aufgebracht.

Bei einem bevorzugten Verfahren wird eine Ladung der Oberfläche und insbesondere eine statische Ladung der Oberfläche durch oben erwähnte Vorrichtung vermindert und bevorzugt im Wesentlichen entfernt.

Bei einem weiteren bevorzugten Verfahren wird die besagte Oberfläche nach der Beaufschlagung mit ionisierter Luft lackiert. Besonders bevorzugt erfolgt dieses Lackieren innerhalb von 20 Minuten, bevorzugt innerhalb von 15 Minuten, bevorzugt innerhalb von zehn Minuten, bevorzugt innerhalb von acht Minuten, bevorzugt innerhalb von 5 Minuten und besonders bevorzugt unmittelbar nach dem Beaufschlagen der Oberfläche mit ionisierter Luft.

Bei einem weiteren bevorzugten Verfahren wird zum Ionisieren der Luft eine Wechselspannung verwendet. Besonders bevorzugt weist dabei die Wechselspannung einen zeitlichen Verlauf auf, der aus einer Gruppe von zeitlichen Verläufen ausgewählt ist, welche dreiecksförmige Verläufe, rechtecksförmige Verläufe und sinusförmige Verläufe enthält.

Besonders bevorzugt wird eine Vorrichtung der oben beschriebenen Art verwendet.

Besonders bevorzugt wird die oben beschriebene Vorrichtung derart gehalten, dass zwischen einem Luftaustritt der Luft aus der Vorrichtung und der zu beaufschlagenden Oberfläche ein Abstand von mehr als 10 cm besteht.

Besonders bevorzugt handelt es sich bei den zu bearbeitenden Oberflächen um Oberflächen von Kraftfahrzeugen. Insbesondere handelt es sich um zu lackierende Oberflächen von Kraftfahrzeugen. Besonders bevorzugt werden die Oberflächen nach der Beaufschlagung mit ionisierter Luft lackiert.

Weitere Vorteile und Ausführungsformen ergeben sich aus den beigefügten Zeichnungen: Darin zeigen:
- Fig. 1: Eine schematische Darstellung einer erfindungsgemäßen Vorrichtung;
- Fig. 2: Eine Darstellung der Ionisierungseinheit der in Figur 1 gezeigten Vorrichtung;
- Fig. 3: Eine Detailansicht der in Figur 2 gezeigten Ionisierungseinheit;
- Fig. 4: eine Darstellung einer weiteren Ausgestaltung der erfindungsgemäßen Vorrichtung;
- Fig. 5: eine Schnittdarstellung der in Fig. 4 gezeigten Vorrichtung;
- Fig. 6: einen Spannungsverlauf einer Ausgangsspannung zum Ionisieren in einer ersten Ausführungsform;
- Fig. 7: einen Spannungsverlauf einer Ausgangsspannung zum Ionisieren in einer zwiten Ausführungsform;
- Fig. 8: eine schematische Darstellung zur Veranschaulichung der Spannungserzeugung;
- Fig. 9: ein Schaltbild einer Wechselspannungswandlereinrichtung.

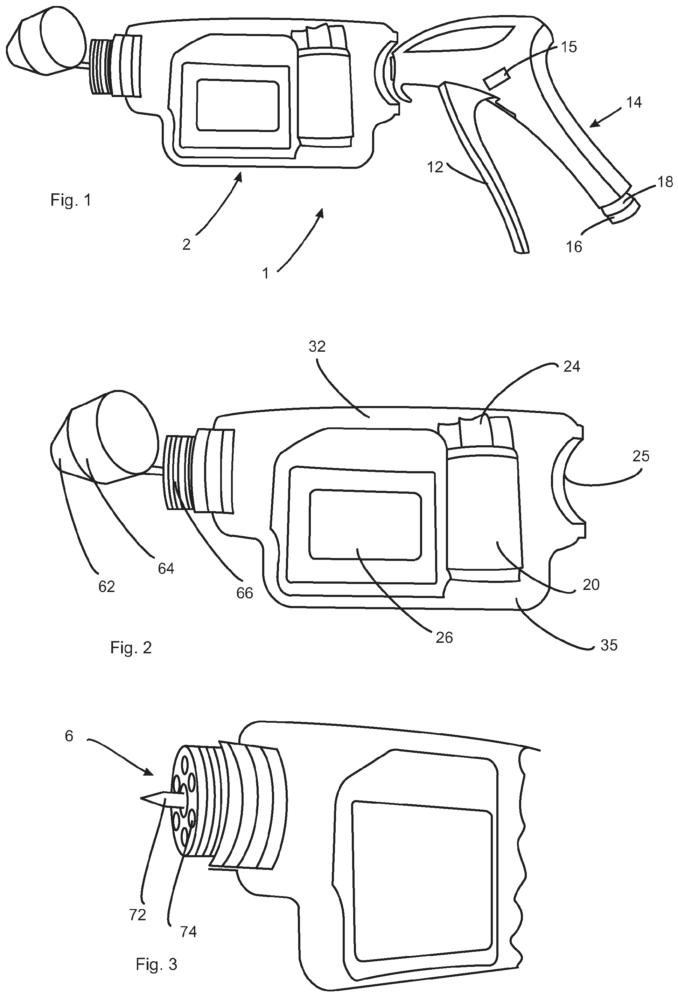

Figur 1 zeigt eine schematische Darstellung einer erfindungsgemäßen Vorrichtung 1 zum Ionisieren von Luft. Diese Vorrichtung weist einen Zuführanschluss 16 auf, über welchen der Vorrichtung Druckluft zugeführt werden kann. Das Bezugszeichen 18 kennzeichnet eine Druckregeleinheit, welche insbesondere manuell verstellbar ist, um den Luftdruck zu verändern.

Das Bezugszeichen 14 kennzeichnet einen Grundkörper und das Bezugszeichen 12 ein Auslöseelement, wie etwa einen Taster oder einen Schalter, mit dem ein Benutzer die Druckluftausgabe steuern kann. Im Inneren des Grundkörpers kann sich dabei bevorzugt eine Ventileinrichtung 15 befinden, welche insbesondere auch von dem Taster bzw. Schalter 12 betätigt werden kann.

Die Druckluft gelangt auf diese Weise in die in ihrer Gesamtheit mit 2 bezeichnete Ionisierungseinheit.

Figur 2 zeigt eine detailliertere Darstellung der Ionisierungseinheit 2. Diese weist einen Zuführanschluss 25 auf. Über diesen wird der Ionisierungseinheit Druckluft zugeführt. Die lonisierungseinheit 2 kann in ihrer Gesamtheit insbesondere über eine Schraubverbindung an dem Grundkörper 14 angeordnet sein.

Das Bezugszeichen 20 kennzeichnet eine Energiewandlereinrichtung. Diese Energiewandlereinrichtung weist ein Turbinenrad 24 auf, welches durch den Luftstrom der unter Druck stehenden Luft angetrieben werden kann. In der Figur würde sich dieses Turbinenrad um eine vertikale Achse drehen. Die Energiewandlereinrichtung kann ein Piezoelement aufweisen, welches diese mechanische Bewegung des Turbinenelements bzw. die von dem Turbinenelement erzeugte mechanische Bewegung in eine elektrische Wechselspannung umsetzt. Daneben könnte jedoch die Energiewandlereinrichtung 20 auch eine Dynamoeinheit aufweisen, welche Wechselspannung erzeugt.

Die von der Energiewandlereinrichtung 20 erzeugte Wechselspannung wird an eine Hochspannungserzeugungseinheit 26 ausgegeben. Diese Hochspannungserzeugungseinheit 26 kann dabei insbesondere eine Transformatoreinheit aufweisen, welche die Wechselspannung hochtransformiert.

Das Bezugszeichen 32 kennzeichnet einen Kanal bzw. einen Leitungsbereich, der zum Leiten bzw. Führen der Druckluft dient. Ausgehend von diesem Kanal 32 gelangt die Luft insbesondere zu der in ihrer Gesamtheit mit 6 bezeichneten Auslasseinrichtung. Bei dieser Auslasseinrichtung kennzeichnet das Bezugszeichen 66 ein Gewinde, auf welches eine Auslassdüse 62 aufschraubbar ist. Diese Düse 62 ist dabei bevorzugt aus einem stromleitenden Material und insbesondere einem Metall gefertigt. Das Bezugszeichen 64 kennzeichnet einen Grundkörper der Ausgabeeinrichtung.

Figur 3 zeigt eine Darstellung der Ausgabeeinrichtung 6 der erfindungsgemäßen Vorrichtung. Man erkennt, dass hier eine Vielzahl von Öffnungen 74 vorgesehen ist, welche in Umfangsrichtung um eine Nadelelektrode 72 angeordnet sind. Durch diese Öffnungen 74 kann die Druckluft ausgeleitet werden und wird damit zwischen zwei Elektroden ionisiert. Eine dieser Elektroden wird dabei durch die Oberfläche 62 bzw. 64 gebildet und die andere Elektrode wie erwähnt durch die Nadel 72. Damit kann die Luft wie oben erwähnt durch das elektrische Feld zwischen dieser Nadelspitze 72 und den Bereichen 62 und 64 ionisiert werden.

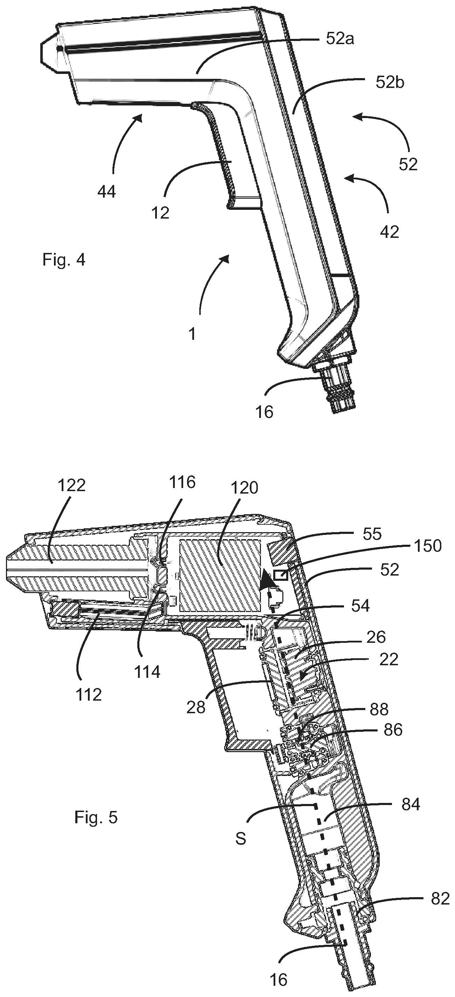

Fig. 4 zeigt eine weitere Ausgestaltung einer erfindungsgemäßen Vorrichtung 1. Diese weist ein Gehäuse bzw. einen äußeren Gehäuseteil 52 auf. Die Vorrichtung weist dabei einen Griffabschnitt 42 auf, der von einem Nutzer gehalten werden kann sowie einen Ausgabeabschnitt, über welchen das gasförmige Medium ausgegeben werden kann.

Das Bezugszeichen 16 kennzeichnet den Zuführanschluss, über welchen der Vorrichtung ein gasförmiges Medium und insbesondere (Druck)Luft zugeführt werden kann. Bevorzugt ist das äußere Gehäuseteil 52 zweiteilig ausgebildet und weist einen Gehäusekörper 52a und einen Gehäusedeckel 52b auf. Diese können bevorzugt an und/oder durch den Zuführanschluss oder ein Element dieses Zuführanschlusses miteinander verbunden sein.

Fig. 5 zeigt eine Schnittdarstellung der in Fig. 4 gezeigten Vorrichtung. Über den Zuführanschluss wird bevorzugt der Vorrichtung 1 Druckluft zugeführt. Der Zuführanschluss ist dabei bevorzugt an dem Griffabschnitt angeordnet. Das Bezugszeichen 82 kennzeichnet dabei eine Kupplung bzw. einen Adapter, an welche(n) die Luftzufuhr angeschlossen werden kann.

Das Bezugszeichen 84 kennzeichnet ein optional vorhandenes Luftreservoir, welchem die Druckluft zugeführt wird. Daneben kann die Vorrichtung bevorzugt auch (nicht gezeigte) Filtereinrichtungen zum Filtern der eintretenden Luft aufweisen, wie etwa Hepa-Filter.

Das Bezugszeichen 86 kennzeichnet ein schaltbares Ventil, welches bevorzugt von einer Feder in eine geschlossene Stellung gedrängt wird und welches bevorzugt mittels des in Fig. 4 gezeigten Auslöseelements geschaltet und somit geöffnet werden kann.

Auf diese Weise gelangt die Druckluft zu der mit dem Bezugszeichen 22 gekennzeichneten Generatoreinrichtung. Diese Generatoreinrichtung 22 weist einen Stator 28 und einen gegenüber diesem Stator drehbaren Rotor 22 auf. Dieser Rotor wird durch die Druckluft zur Drehung angetrieben.

Das Bezugszeichen S kennzeichnet die Hauptströmungsrichtung der innerhalb des Gehäuses bzw. des Griffabschnitts 42 strömenden Luft. Die Drehachse des Rotors steht bei der in Fig. 5 gezeigten Ausgestaltung im Wesentlichen senkrecht zu der Hauptströmungsrichtung S.

Damit treibt der pneumatische Druck die Generatoreinrichtung an, wobei durch die Ausgestaltung der Generatoreinrichtung als Außenläufermaschine einen verbesserten Durchgang für die Druckluft bietet.

Der in der Generatoreinrichtung erzeugte Strom wird zu einer Eingangsleistung für die Vorrichtung und/oder einer Eingangsplatine und/oder einem Transformator 120 zugeführt. Wenn die Eingangsleistung eine bestimmte Spannung überschreitet, wird diese Spannung an die beiden Pins 114 und 116 (oder an einen der beiden Pins) gelegt und diese ionisieren die durchtretende Luft. Bevorzugt ist an die Pins 114 und 116 eine Wechselspannung ausgebbar. Es ist dabei auch und bevorzugt möglich, dass einer der beiden Pins geerdet ist.

Das Bezugszeichen 150 kennzeichnet eine Wechselspannungswandlereinrichtung, welche einen vorgegebenen (zeitlichen) Verlauf der ausgegebenen Spannung, wie etwa einen dreieckförmigen Verlauf erzeugt. Bevorzugt ist dieser vorgegebene zeitliche Verlauf änderbar.

Bevorzugt ist beispielsweiose auch eine Größe der ausgegebenen Spannung veränderbar.

Dabei ist bevorzugt der Transformator in dem Ausgabeabschnitt 44 der Vorrichtung 1 angeordnet. Das Bezugszeichen 122 kennzeichnet die eigentliche Düse, durch welche die Druckluft nach außen abgegeben wird.

Das Bezugszeichen 112 kennzeichnet eine Beleuchtungseinrichtung, die bevorzugt ebenfalls von dem Transformator 120 und damit auch von der Generatoreinrichtung 22 elektrisch versorgt wird. Diese Beleuchtungseinrichtung weist bevorzugt eine Linse und besonders bevorzugt eine Zylinderlinse auf.

Bevorzugt ist eine Strahlungsrichtung der Beleuchtungseinrichtung 112 geringfügig gegenüber der Strömungsrichtung der Luft innerhalb der Düse 122 geneigt, so dass bei einem vorgegebenen Abstand zwischen der Vorrichtung und einer zu beaufschlagenden Oberfläche in guter Näherung der Lichtkegel der von der Beleuchtungseinrichtung ausgegebenen Strahlung und der Kegel der austretenden Luft zusammenfallen.

So kann beispielsweise die Vorrichtung auf einen bestimmten Abstand der Vorrichtung von der zu beaufschlagenden Oberfläche kalibriert bzw. eingestellt sein, etwa einen Sprühabstand zwischen 10cm und 20cm.

Daneben wäre es auch möglich, dass die Strahlrichtung, in der die Beleuchtungseinrichtung 112 ihre Strahlung ausgibt, verändert werden kann, um so die Abstände, in denen der Lichtkegel der von der Beleuchtungseinrichtung ausgegebenen Strahlung und der "Sprühkegel" der von der Düseneinrichtung ausgegebenen Luft im Wesentlichen zusammenfallen. Auf diese Weise kann dem Benutzer mittels der Beleuchtungseinrichtung der Oberflächenbereich angezeigt werden, der gerade von der ionisierten Luft beaufschlagt wird.

Das Bezugszeichen 52 kennzeichnet wiederum das Außengehäuse der Vorrichtung, innerhalb dessen bevorzugt ein Innengehäuse 54 angeordnet ist, welches besonders bevorzugt auch wenigstens eine Komponente, bevorzugt mehrere Komponenten der Vorrichtung (wie etwa die Generatoreinrichtung oder den Transformator) trägt. Auch diese Weise kann in besonders vorteilhafter Weise eine elektrische Isolierung erreicht werden. Bevorzugt sind auch die beiden die Ionisierung bewirkenden Pins in diesem inneren Gehäuse angeordnet.

Das Bezugszeichen 55 kennzeichnet eine Warneinrichtung wie etwa eine Warnleuchte. Diese kann bevorzugt als mehrfarbige Beleuchtung ausgeführt sein, welche durch unterschiedliche Lichtausgaben Hinweise an den Benutzer ausgeben kann. So kann beispielsweise ein Blinken dieser Warneinrichtung anzeigen, dass ein zu geringer Druck an dem Anschluss 16 anliegt, ein durchgehend beispielsweise grünes Leuchten kann anzeigen, dass der Druck in einem gewünschten Bereich liegt und ein rotes Leuchten könnte einen zu hohen Druck anzeigen.

Dabei kann weiterhin die Vorrichtung eine Messeinrichtung aufweisen, welche wenigstens einen Messwert erfasst, der für einen anliegenden Luftdruck charakteristisch ist. So könnte beispielsweise direkt der anliegende Druck über eine Druckmesseinrichtung bestimmt werden. Auch könnte über einen Durchflussmesser eine Strömungsgeschwindigkeit der durchströmenden Luft gemessen werden oder aber es könnte aufgrund der generierten Spannungen und/oder Ströme auf den Luftdruck oder die Strömungsgeschwindigkeit geschlossen werden.

Bevorzugt weist die Vorrichtung eine Prozessoreinrichtung (nicht gezeigt), welche auf Basis des von der Messeinrichtung ausgegebenen Messwertes ein Signal an die Warneinrichtung ausgibt.

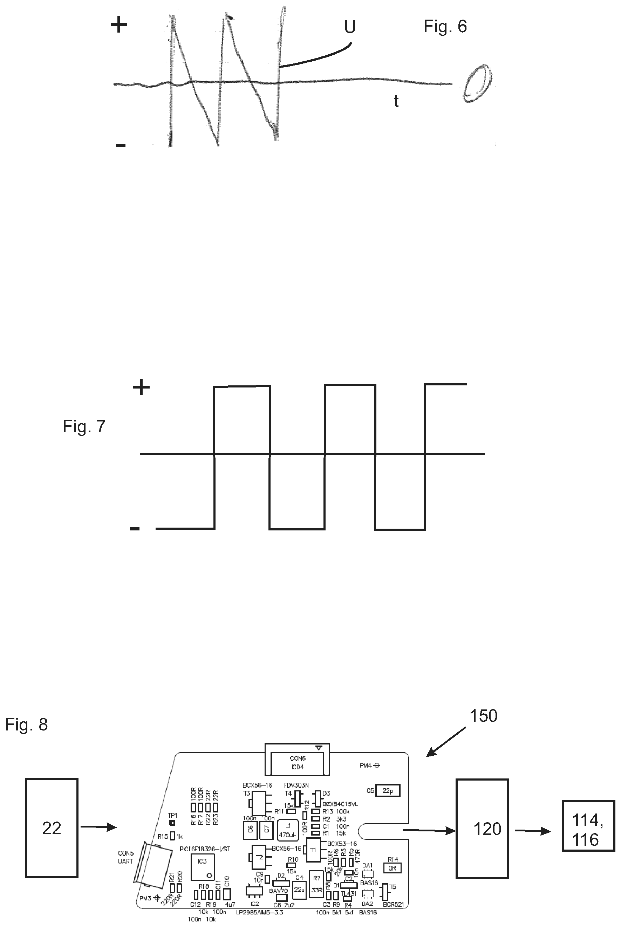

Fig. 6 zeigt einen Spannungsverlauf der Spannung mit welcher die Ionisiereinheit und/oder deren Elektroden beaufschlagt werden. Man erkennt, dass diese Spannung einen periodischen Verlauf annimmt und bei der in Fig. 6 gezeigten besonders bevorzugten Ausgestaltung einen sägezahnartigen Verlauf annimmt.

Dabei steigt die Spannung ausgehend von einem negativen Potential zunächst schlagartig auf ein positives Potential an und sinkt dann wieder, insbesondere mit konstanter Steigung auf das negative Potential zurück.

Bevorzugt stimmen das positive (Spannungs)Potential gegenüber 0 und/oder gegenüber der Erdung und das negative (Spannungs)Potential miteinander überein oder weichen um nicht mehr als 20% und bevorzugt um nicht mehr als 10% voneinander ab.

Fig. 7 zeigt einen weiteren möglichen Spannungsverlauf, hier in Form eines Rechteckprofils.

Fig. 8 zeigt eine schematische Darstellung der Spannungserzeugung. Die Energiewandlereinrichtung 22 erzeugt aus der Druckluft eine (Wechsel)spannung. Diese Wechselspannung wird als Eingangssignal bzw. Eingangsspannung einer Wechselspannungswandlereinrichtung 150 zugeführt.

Diese Wechselspannungswandlereinrichtung erzeugt ein Ausgangssignal etwa in Form einer Dreiecksspannung bzw. mit einem dreiceksförmigen Verlauf. Diese Dreiecksspannung wird bevorzugt durch eine Transportmatoreinrichtung, welche jedoch bevorzugt einen Bestandteil der Wechselspannungswandlereinrichtung bildet, transformiert.

Die so erzeugte Wechselspannung wird den Elektroden 114, 116 zum Ionisieren der Druckluft zugeführt.

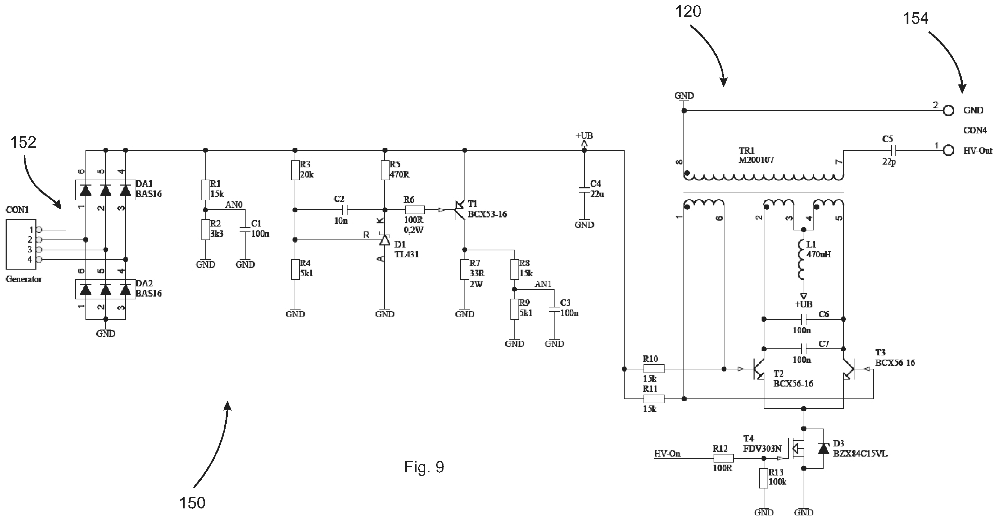

Fig. 9 zeigt eine Schaltbilddarstellung einer Wechselspannungswandlereinrichtung 150. Diese weist einen Spannungseingang 152 auf, über den der Wechselspannungswandlereinrichtung die Eingangsspannung, insbesondere von der Energiewandlereinrichtung zugeführt wird.

Das Bezugszeichen 154 kennzeichnet einen Ausgang, über welchen die von der Wechselspannungswandlereinrichtung erzeugte Wechselspannung ausgegeben wird, insbesondere an die Elektrode(n).

Bevorzugt wird die Eingangsspannung gleichgerichtet und geglättet. Bevorzugt wird mittels zweier Transistoren eine Spannung mit einem gewünschten zeitlichen Profil erzeugt. Diese wird mittels einer Transformatoreinrichtung transformiert und an den Ausgang 154 ausgegeben.

Es wird darauf hingewiesen, dass sämtliche Merkmale, welche unter Bezugnahme auf das Verfahren beschrieben wurden, in entsprechender Weise auch für die Vorrichtung offenbart sind, was insbesondere bedeutet, dass die entsprechende Vorrichtung Einrichtungen aufweist, welche zur Durchführung der jeweiligen Verfahren geeignet und bestimmt sind. Weiterhin sind auch Merkmale, welche unter Bezugnahme auf die Vorrichtung beschrieben wurden entsprechend für das oder die Verfahren anwendbar. Dies bedeutet, dass die Verfahren unter Verwendung der entsprechenden Vorrichtungsmerkmale ausgeführt werden.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer in einzelnen oder in unterschiedlichen Figuren gezeigter Merkmale ergeben können.

## Patentansprüche

1. Vorrichtung (1) zum Beaufschlagen von Oberflächen und insbesondere von Kraftfahrzeugoberflächen mit ionisierter Luft mit einer Zuführanschluss, um der Vorrichtung (1) Druckluft zuzuführen, mit einer Ionisierungseinheit (2), welche dazu geeignet und bestimmt ist, wenigstens einen Anteil der über den Zuführanschluss (2) zugeführten Luft zu ionisieren und mit einer Ausgabeeinrichtung (6), welche die ionisierte Luft auf die Oberfläche ausgibt, wobei die Ionisierungseinheit (2) eine Energiewandlereinrichtung (22) aufweist, welche eine Bewegungsenergie des Luftstroms wenigstens teilweise in eine elektrische Energie umwandelt, wobei die Vorrichtung (1) einen Griffabschnitt (42) aufweist, welcher zum Halten der Vorrichtung dient sowie einen Ausgabeabschnitt (44), der zum Ausgeben der ionisierten Luft dient,
**dadurch gekennzeichnet, dass**
die Ionisiereinheit (2) dazu geeignet und bestimmt ist, die Luft mit einer zeitlich variierenden Spannung zu ionisieren.

2. Vorrichtung nach Anspruch 1,
**dadurch gekennzeichnet, dass**
die Ionisierungseinheit (2) dazu geeignet und bestimmt ist, die Luft wenigstens zeitweise mit einer positiven Spannung und wenigstens zeitweise mit einer negativen Spannung zu ionisieren und/oder die Ionisierungseinheit (2) einen Wechselspannungsgenerator (22, 120, 150) aufweist, der eine Wechselspannung erzeugt.

3. Vorrichtung nach wenigstens einem der vorangegangenen Ansprüche,
**dadurch gekennzeichnet, dass**
der Wechselspannungsgenerator eine periodisch verlaufende Wechselspannung erzeugt und die periodisch verlaufende Wechselspannung einen sinusförmigen Verlauf oder einen sägezahnförmigen Verlauf oder einen rechtecksförmigen Verlauf oder Kombinationen hieraus aufweist.

4. Vorrichtung nach wenigstens einem der vorangegangenen Ansprüche,
**dadurch gekennzeichnet, dass**
die Energiewandlereinrichtung wenigstens abschnittsweise in den Griffabschnitt integriert ist.

5. Vorrichtung nach wenigstens einem der vorangegangenen Ansprüche,
**dadurch gekennzeichnet, dass**
der Wechselspannungsgenerator eine periodisch verlaufende Wechselspannung erzeugt und diese periodische Wechselspannung eine Frequenz aufweist, welche höher ist als 100Hz, bevorzugt höher als 200Hz, bevorzugt höher als 400Hz, bevorzugt höher als 800Hz und bevorzugt höher als 1000Hz und besonders bevorzugt höher als 2000Hz und/oder bevorzugt niedriger als 50kHz, bevorzugt niedriger als 40kHz, bevorzugt niedriger als 30kHz, bevorzugt niedriger als 20kHz und/oder der Wechselspannungsgenerator oder ein von dem Wechselspannungsgenerator gespeister Transformator eine periodisch verlaufende Wechselspannung erzeugt und diese periodische Wechselspannung größer ist als 100V, bevorzugt größer als 200V, bevorzugt größer als 400V, bevorzugt größer als 600V, bevorzugt größer als 1kV, bevorzugt größer als 2kV, bevorzugt größer als 4kV und/oder diese periodische Wechselspannung kleiner ist als 40kV, bevorzugt kleiner als 30kV, bevorzugt kleiner als 20kV, bevorzugt kleiner als 15kV.

6. Vorrichtung (1) nach wenigstens einem der vorangegangenen Ansprüche,
**dadurch gekennzeichnet, dass**
die Energiewandlereinrichtung die Ionisierungseinheit wenigstens mittelbar mit elektrischer Energie versorgt.

7. Vorrichtung (1) nach wenigstens einem der vorangegangenen Ansprüche,
**dadurch gekennzeichnet, dass**
der Wechselspannungsgenerator (22, 150) eine Wechselspannungswandlereinrichtung (22, 150) aufweist, welche eine Eingangsspannung und insbesondere eine Eingangswechselspannung mit einem ersten vorgegebenen zeitlichen Verlauf in eine Wechselausgangsspannung mit einem zweiten vorgegebenen Verlauf aufweist, wobei der zweite vorgegebene zeitliche Verlauf von dem ersten vorgegebenen zeitlichen Verlauf abweicht und/oder der Wechselspannungsgenerator (150) von der Energiewandlereinrichtung gespeist wird und/oder ein Ausgangssignal und/oder eine Ausgangsspannung der Energiewandlereinrichtung wenigstens mittelbar dem Wechselspannungsgenerator als Eingangssignal zugeführt wird.

8. Vorrichtung nach wenigstens einem der vorangegangenen Ansprüche 6 - 7,
**dadurch gekennzeichnet, dass**
die Wechselausgangsspannung einem lonisierelement und/oder wenigstens einer Elektrode zugeführt wird, welche die Luft ionisiert.

9. Vorrichtung (1) nach wenigstens einem der vorangegangenen Ansprüche,
**dadurch gekennzeichnet, dass**
die Vorrichtung (1) eine Erdungseinrichtung aufweist, um die Vorrichtung in einem Betrieb elektrisch zu erden und/oder die Ausgabeeinrichtung (6) ein elektrisch leitendes Anschlussmittel aufweist, um die Ausgabeeinrichtung mit einer Erdungsleitung zu verbinden.

10. Vorrichtung (1) nach wenigstens einem der vorangegangenen Ansprüche,
**dadurch gekennzeichnet, dass**
die Vorrichtung eine Anschlussleitung aufweist, mittels derer der Ausgabeeinrichtung (6) Druckluft zuführbar ist, wobei diese Anschlussleitung bevorzugt auch dazu geeignet und bestimmt ist, um wenigstens ein Element der Ausgabeeinrichtung (6) und insbesondere eine Elektrode elektrisch zu erden.

11. Vorrichtung (1) nach wenigstens einem der vorangegangenen Ansprüche,
**dadurch gekennzeichnet, dass**
wenigstens ein Parameter der für die von der Ionisierungseinheit zur Ionisierung verwendete Spannung charakteristisch ist veränderbar ist, wobei bevorzugt dieser Parameter aus einer Gruppe von Parametern ausgewählt ist, welche eine Frequenz der Spannung, eine maximale positive Spannung, eine maximale negative Spannung, einen zeitlichen Verlauf der Spannung, eine Symmetrie der Spannung bezüglich eines vorgegebenen Potenzials und dergleichen enthält.

12. Vorrichtung (1) nach wenigstens einem der vorangegangenen Ansprüche,
**dadurch gekennzeichnet, dass**
die Vorrichtung (1) eine Sensoreinrichtung zur Erfassung eines Luftdrucks oder eines Luftstroms aufweist.

13. Vorrichtung (1) nach wenigstens einem der vorangegangenen Ansprüche,
**dadurch gekennzeichnet, dass**
die Vorrichtung (1) eine Beleuchtungseinrichtung zur Beleuchtung der zu bearbeitenden Oberfläche und insbesondere Kraftfahrzeugoberfläche aufweist, wobei bevorzugt die Beleuchtungseinrichtung eine Linseneinrichtung aufweist, wobei bevorzugt diese Beleuchtungseinrichtung wenigstens ein optisch beugendes Element aufweist, welches bewirkt, dass eine von der Beleuchtungseinrichtung beleuchtete Oberfläche der Oberfläche einen von einem kreisförmigen Querschnitt abweichenden Querschnitt und insbesondere einen elliptischen Querschnitt aufweist und/oder bevorzugt die Beleuchtungseinrichtung von der Energiewandlereinrichtung mit elektrischer Energie versorgbar ist.

14. Vorrichtung (1) nach dem vorangegangenen Anspruch,
**dadurch gekennzeichnet, dass**
die Vorrichtung eine Abstandserfassungseinrichtung aufweist, welche einen Abstand zwischen der Vorrichtung und der zu beaufschlagenden Oberfläche bestimmt.

15. Vorrichtung (1) nach wenigstens einem der vorangegangenen Ansprüche,
**dadurch gekennzeichnet, dass**
die Vorrichtung wenigstens ein mit einer Spitze ausgebildetes lonisierelement und bevorzugt wenigstens zwei mit einer Spitze ausgebildete lonisierelemente aufweist, welche an diesen lonisierelementen vorbeiströmende Luft ionisiert.
